# EUROPEAN PATENT APPLICATION

(11) **EP 2 450 024 A2**
(43) Date of publication of application: **09.05.2012**
(21) Application number: 11187940.9
(22) Date of filing: 04.11.2011
(51) Int. Cl.: A61K 6/00, A61K 6/083

(54) **Dental material with color indicator and metods of using same**

(30) Priority: 03.11.2011 US 288253; 04.11.2010 US 410171 P; 05.11.2010 US 410443 P; 05.11.2010 US 410565 P
(71) Applicant: Kerr Corporation, Orange, CA 92857 (US)
(72) Inventor: Qian, Xuejun, Foothill Ranch, California 92610 (US); Chen, Xiangxu, Diamond Bar, California 91765 (US)
(74) Representative: Findlay, Alice Rosemary

(57) **Abstract**

A dental material is provided that exhibits a color change upon or after mixing or contacting components of the composition. The composition includes an acidic component in one part, and a basic component in another part that upon contact with the acidic component initiates a chemical reaction thereof that alters the pH of the composition. An acid or base sensitive color change agent is present in the composition that registers the change in pH by changing color as the pH changes.

## Description

The present invention relates to dental materials and, in certain specific embodiments, to dental cements and bonding agents.

Hand mixing is widely used with dental materials, for example with the current dental cement pastes, and mixing efficiency is always a concern. It is difficult for the clinician to tell when the pastes are fully mixed. In addition, during the clean-up stage after the cement is applied to the tooth or other dental structure, it can be difficult to discern where the excess material is that needs to be removed, particularly since the material is designed to blend in or coordinate with the tooth color. Yet another issue, with bonding agents, is the difficulty in seeing where the bonding agent has been placed. Commercial products do not adequately address these issues.

This disclosure is directed to a dental composition that is capable of undergoing a color change. To that end, the dental composition comprises a first part containing an acidic component, a second part containing a basic component, and at least one color change agent in the first and/or the second part that effects a change in color in the dental composition upon an increase or decrease in pH.

This disclosure is also directed to methods of applying the dental composition to a dental structure. In one method, a first part containing the acidic component is mixed with a second part containing the basic component to form a mixed dental composition in which the neutralization reaction takes place. The mixed dental composition is then applied to the dental structure, and the color change agent(s) present in one of the two parts effects a change in color in the mixed dental composition upon an increase in pH from the neutralization reaction. In another method, a first composition containing the acidic component, water and the at least one color change agent is applied to the dental structure, wherein the first composition is self-etching and/or self-adhesive to the dental structure. A second composition containing the basic component is thereafter applied to the dental structure in contact with the first composition, wherein the second composition is non-self-etching and non-self-adhesive to the dental structure and initiates a neutralization reaction with the acidic component, and the color change agent(s) effects a change in color in the first dental composition upon an increase in pH from the neutralization reaction. The invention will now be further described by way of the following detailed description of preferred embodiments.

The present invention provides a dental material that exhibits a color change upon mixing components of the composition. To that end, the composition includes an acidic component and a basic component, which upon contact, undergo a chemical reaction, such as a neutralization reaction of the acid, that alters the pH of the composition. A color change agent is present in the composition that registers the change in pH by changing color as the pH changes. The color change agent may be an acid sensitive color change agent or a base sensitive color change agent. By way of example, the agent may have a visually observable color, such as a red color, that changes to colorless or a visually non-observable color as the pH changes, for example as the pH increases from a more acidic pH toward a less acidic or neutral or basic pH. The color change (i.e., the disappearance of a visually observable color) may then be indicative of the extent of mixing, i.e., a colorless composition may indicate that the components are fully and/of homogenously mixed, or the extent of hardening, for example.

In one embodiment, the present invention provides a method for applying a dental composition to a dental structure. The method includes a first step of mixing two or more components (or parts or pastes) of a dental composition, wherein one component comprises at least one organic acid and the other component(s) comprises at least one filler that can react with the organic acid. At least one acid sensitive color change agent is included with any one of these two or more components. The method further includes a second step of applying the mixed dental composition onto a dental structure. In accordance with the invention, the color of the mixed composition changes from one visually observable color to a significantly different color or visual lack thereof. The filler itself may be a base component that neutralizes the organic acid in the other component upon contact during mixing, or a different basic component may be present in one or more of the components that are mixed with the component containing the organic acid to initiate the neutralization reaction. Alternatively, a different chemical reaction may be initiated upon contact that effectively decreases the pH, and the color change agent may be base sensitive to thereby change color to register that decrease in pH. The decrease in pH may be from a more basic pH toward a less basic or neutral or acidic pH.

In one embodiment, the organic acid is a polymeric carboxylic acid. In another embodiment, the organic acid comprises at least one ethylenically unsaturated group. In another embodiment, the organic acid comprises at least one polymeric carboxylic acid and at least one organic acid with at least one ethylenically unsaturated group. In another embodiment, the organic acid is a carboxylic acid with at least one ethylenically unsaturated group. In another embodiment, the organic acid is a phosphoric acid or phosphate with at least one ethylenically unsaturated group. In one embodiment, the dental composition comprises at least one ethylenically unsaturated monomer. In one embodiment, the dental composition comprises at least one free radical initiator. In one embodiment, the filler is a fluoroaluminosilicate filler. In one embodiment, the acid sensitive color change agent is penta-methoxy red. In one embodiment, the color change is from a visually observable color to essentially colorless. In one embodiment, the color change is concurrent with the second step of applying the mixed composition to the tooth structure. In another embodiment, the color change occurs after the second step of applying the mixed composition to the tooth structure, and may even be significantly thereafter. In one embodiment, the dental composition is further exposed to a dental curing light.

In another embodiment, the present invention provides a method for applying a dental composition to a dental structure. The method includes a first step of applying a first dental composition to a dental structure, wherein the first dental composition comprises at least one organic acid, water, and at least one acid sensitive color change agent. The first dental composition is self-etching or self-adhesive on the dental structure, such as a tooth structure. The method further includes a second step of applying a second dental composition that is non-self-etching or non-self-adhesive onto at least a portion of the dental structure to effectively cause a visually observable color change in that portion of the dental structure surface also having the self-etching or self-adhering composition applied thereon. The second dental composition contains a basic component that initiates a neutralization reaction when it comes in contact with the organic acid in the first dental composition, which effectively alters the pH, and that change in pH is indicated by the color change due to the presence of the acid sensitive color change agent(s). Alternatively, a different chemical reaction may be initiated upon contact that effectively decreases the pH, and the color change agent may be base sensitive to thereby change color to register that decrease in pH. The decrease in pH may be from a more basic pH toward a less basic or neutral or acidic pH.

In one embodiment, the organic acid is as described above. In one embodiment, the first and/or second dental composition comprises at least one ethylenically unsaturated monomer. In one embodiment, the first and/or second dental composition comprises at least one free radical initiator. In one embodiment, the second dental composition comprises a basic filler, for example a fluoroaluminosilicate filler. In one embodiment, the basic component is an inorganic base. In one embodiment, the basic component is an organic base. In one embodiment, the basic component is a tertiary amine. In one embodiment, the color change is from a visually observable color (other than light yellow) to light yellow. In one embodiment, the color change is from a visually observable color to essentially colorless. In one embodiments, the acid sensitive color change agent is from the group of methyl yellow, methyl red, thymol blue, neutral red and penta-methoxy red. In one embodiment, the color change agent is penta-methoxy red. In one embodiment, the first and/or second dental composition is further exposed to a dental curing light.

In those embodiments in which a second reactive component is mixed with or contacts a first reactive component after the first component is applied to the dental structure, the first dental composition may be applied to the dental structure while having a visually discernible color compared to the dental structure, such that it is easy to see where exactly the material has been applied and any excess material is easily identified for removal. Thereafter, the second dental composition may be applied onto the first to cause the color to change to a non-discernible color, for example, and essentially colorless appearance. In those embodiments where the mixing of the components occurs before the composition is applied to the tooth structure, the color change may happen quickly, for example, to indicate full and thorough mixing, or it may occur slowly or after a period of delay to permit time for the composition to be carefully applied to the dental structure and for any excess material to be removed.

In one embodiment, organic acids include, but are not limited to, phosphoric acid, a phosphoric acid derivative, phosphonic acid, a phosphonic acid derivative, carboxylic acid, carboxylic acid anhydride, sulfonic acid, sulfinic acid, with a derivative being a salt or ester of the respective acid. Examples of these acidic compounds include, but are not limited to, maleic acid, itaconic acid, methacrylic acid, acrylic acid, tartaric acid, ethylenediaminetetraacetic acid (EDTA), EDTA salt, citric acid, a homopolymer or copolymer of an α,β-unsaturated carboxylic acid such as poly(acrylic acid), copolymer of acrylic acid such as poly(acrylic acid-maleic acid) copolymer or poly(acrylic acid-itaconic acid) copolymer or poly(acrylic acid-maleic acid-itaconic acid) copolymer, polymerizable homopolymer or copolymer of an α,β-unsaturated carboxylic acid such as (meth)acrylated poly(acrylic acid), (meth)acrylated poly(acrylic acid) copolymer such as (meth)acrylated poly(acrylic acid-maleic acid) copolymer or (meth)acrylated poly(acrylic acid-maleic acid-itaconic acid) copolymer, maleic anhydride, trimellitic anhydride, 4-META (4-methacrytoxyethyltrimellitic anhydride), any addition product of mono- or di-anhydride compound with a hydroxyalkylmethacrylate compound such as PM-HEMA (addition product of pyromellitic acid anhydride and 2-1-hydroxyethyl methacrylate), PM-GDM (addition product of pyromellitic acid anhydride and glycerol dimethacrylate), BTDA-HEMA (addition product of 3,3',4,4'-benzophononotetracarboxylic dianhydride and hydroxyethyl methacrytate), and PA-HEMA (addition product of phthalic anhydride and hydroxyethyl methacrylate), MA-GDM (addition product of maleic anhydride and glycerol dimethacrylate), sulfuric acid, alkyl sulfonic acid, aromatic sulfonic acid, alkyl sulfinic acid, aromatic sulfinic acid, phosphoric acid, pyrophosphoric acid, monoalkyl phosphate, dialkyl phosphate, aryl alkyl phosphate, aryl phosphate, phenyl-P (phenyl methacryloxyethyl phosphate), GDM-P (glyceryldimethacrylate phosphate), PENTA-P (pentaerithritol triacrylate phosphate), MDP (methacryloyloxydecyl phosphate), HEMA-P (hydroxyethylmethacrylate phosphate), HEA-P (hydroxyethylacrylate phosphate), bis(HEMA)-P (bis(hydroxyethylmethacrylate) phosphate), bis(HEA)-P (bis(hydroxyethylacrylate) phosphate), bis((meth)acryloxypropyl)phosphate and a combination thereof.

In one embodiment, the ethylenically unsaturated group is selected from the groups consisting of acrylate, methacrylate, acrylamide, methacrylamide and vinyl group. Monofunctional monomers include, but are not limited to, methyl (meth)acrylate, ethyl (meth)acrylate, propyl (meth)acrylate, butyl (meth)acrylate, hydroxyethyl (meth)acrylate, hydroxypropyl (meth)acrylate, glycerol mono(meth)acrylate, polyethyleneglycol mono-(meth)acrylate, polypropyleneglycol mono-(meth)acrylate, polytetramethyleneglycol mono-(meth)acrylate, or a combination thereof.

In one embodiment, the ethylenically unsaturated monomer(s) have at least two ethylenically unsaturated groups. Multi-functional monomers can form crosslinking network with polymerization and make the dental adhesive layer durable. Multi-functional monomers include, but are not limited to, glycerol di(meth)acrylate, glycerol tri(meth)acrylate, 2,2-bis[4-(2-hydroxy-3 -methacryloylpropoxy)-phenyl]-propane (BisGMA), urethane di(meth)acrylate, ethoxylated bisphenol A dimethacrylate (EBPADMA-n where n-total number of moles of ethylene oxide in the molecule, as only one example, n=2-20 units), ethylene glycol di(meth)acrylate, neopentyl glycol di(meth)acrylate, tetraethylene glycol di(meth)acrylate, triethytene glycol di(meth)acrylate, diethylene glycol di(meth)acrylate, 1,3-butylene glycol di(meth)acrylate, cyclohexane dimethanol di(meth)acrylate, 1,6-hexanediol di(meth)acrylate, 1,4-butanediol dimethacrylate, propoxylated glyceryl tri(meth)acrylate, polyethyleneglycol di-(meth)acrylate, polypropyleneglycol di-(meth)acrylate, polytetramethyleneglycol di-(meth)acrylate, hexanediol di(meth)acrylate, trimethylolpropane tri(meth)acrylate, ethoxylated trimethylolpropane tri(meth)acrylate, tris (2-hydroxy ethyl) isocyanurate tri(meth)acrylate, pentaerythritol di(meth)acrylate, pentaerythritol tri(meth)acrylate, pentaerythritol tetra(meth)acrylate, dipentaerythritol penta(meth)acrylate, multifunctional aliphatic urethane (meth)acrylate, multifunctional aromatic urethane (meth)acrylate or mixtures thereof. Exemplary multi-functional monomers include glycerol di(meth)acrylate, glycerol tri(meth)acrylate, 2,2-bis[4-(2-hydroxy-3-methacryloylpropoxy)-phenyl]-propane (BisGMA), urethane di(meth)acrylate, trimethylolpropane tri(meth)acrylate, ethoxylated trimethylolpropane tri(meth)acrylate, and dipentaerythritol penta(meth)acrylate.

In one embodiment, the composition comprises at least one polymerization initiator for initiating polymerization of the composition and causing hardening of the composition. The amount of polymerization initiator(s) may be in the range of 0.01 to 10% by weight, for example, in the range of 0.05 to 8% by weight, and by further example, in the range of 0.1 to 5% by weight. In one embodiment, the polymerization initiator is a re-dox free radical initiator.

In one embodiment, the initiator is a photo-initiator that initiates polymerization of the composition through light activation with a dental curing light capable of generating ultraviolet and/or visible light. A photo-initiator usually comprises a photo-sensitizer and a reducing agent. Photo-initiators/sensitizers include, but are not limited to, camphorquinone (CQ), phenathrenequinone, 4,4'-bis(dimethylamino)benzophenone and 4,4'-bis(diethylamino)benzophenone. CQ has absorption of both ultraviolet and visible lights, and therefore, it is an exemplary photo-initiator. Amines, especially tertiary amines, can be used as reducing agents for CQ to co-initiate free radical polymerization. Possible tertiary amines include, but are not limited to, ethyl-4-(N,N-dimethylamino) benzoate (EDMAB), 2-ethylhexyl-4-(N,N-dimethylamino) benzoate (ODMAB), 4-dimethylamino-benzophenone (DMABP), p-dimethylamino benzoic acid (DMABA), p-(dimethylamino) benzonitrile (DMABCN), p-(dimethylamino) benzaldehyde, 4'-morpholino-acetophenone, 4'-morpholinobenzophenone, p-(dimethylamino) acetophenone, 4,4'-bis(dimethylamino)benzophenone, 4,4'-bis(diethylamino) benzophenone and dimethylaniline. Among these tertiary amines, EDMAB, ODMAB, DMABP, DMABA and DMABCN are exemplary. Other reducing agents for camphorquinone include but are not limited to chemical compounds with urethane and benzhydyl groups.

Phosphine oxides, including mono-acyl and multi-acyl phosphine oxide, can also be used as photoinitiators. Phosphine oxides can initiate free radical polymerizations by themselves under ultraviolet and/or visible irradiations generated by a typical dental curing device. Examples of phosphine oxides include, but are not limited to, bis(2,4,6-trimethylbenzoyl)-plienyl phosphine oxide (Irgacure 819, Ciba Specialty Chemicals, Basel, Switzerland), bis(2,6-dimethoxybenzoyl)-(2,4,4-trimethylpentyl) phosphine oxide (CGI 403, Ciba Specialty Chemicals) ethyl 2,4,6-trimethylbenzoyl-phenyl phosphine oxide (LUCIRIN LR8893X, BASF Corp., Charlotte, N.C.). Combinations of two or more phosphine oxides are also advantageous. Examples of combinations of phosphine oxides include, but are not limited to, a 50:50 by weight mixture of 2,4,6-trimethylbenzoyl-diphenyl phosphine oxide and 2-hydroxy-2-methyl-1-phenylpropane-1-one (DAROCUR 4265, Ciba Specialty Chemicals).

Fluoron and pyronin derivatives can also initiate free radical polymerizations together with amines and iodonium synergists under UV and/or visible irradiations generated by a typical dental curing device. Examples of fluoron and pyronin derivatives include, but are not limited to, 5,7-diiodo-3-butoxy-6-fluorone (H-Nu 470, Spectra Group Ltd., Millbury, Ohio). Other examples of fluoron and pyronin derivatives that can initiate free radical polymerizations are described in, but not limited to, U.S. Pat. No. 5,623,080 (Neckers and Shi) and U.S. Pat. No. 5,451,343 (Neckers and Shi).

Fillers can also be incorporated into the compositions of the present invention. Fillers enhance mechanical properties of the composition, reduce polymerization shrinkage, improve rheological and handling properties of the composition, and increase radiopacity of the composition for ease in detection of gaps or voids. Examples of fillers include, but are not limited to, inorganic metal, salt, oxide, nitride, silicate glass, aluminosilicate glass, aluminoborosilicate glass, fluoroaluminosilicate glass, quartz, colloidal silica, fumed silica, precipitated silica, zirconia silica, polymeric filler and /or polymerized composite fillers with inorganic particles. Fumed silica, colloidal silica and precipitated silica may be particularly useful in the inventive compositions. Examples of colloidal and fumed silicas include, but are not limited to, Aerosil series and AERODISP series (both from Degussa, Ridgefield Park, NJ) and Cab-O-Sil series (Cabot Corp., Tuscola, IL). Aerosil series include, but are not limited to, Aerosil 150, 200, 300, 380, R202, R805, R972, OX-50, OX-130 and OX200 silica. AERODISP series include, but are not limited to, AERODISP W1714, W1824, W1836, W630, W7512S and W7520, all of which are water-based dispersions. Cab-O-Sil series include, but are not limited to, Cab-O-Sil M5, LM-150, TS-720, TS-610 and TS-530. The filler also includes nanoparticles, such as those obtained through a sol-gel process. Examples include those disclosed in U.S. Patent Nos. 4,567,030 (Yuasa et al.) and 5,609,675 (Noritake and Yuasa). Mixtures of different fillers can be used. For inorganic fillers, the surface of the filler may be treated or coated with a coupling agent, such as gamma-methacryloyloxypropyltrimethoxy-silane (MPTMS), that enhances the interfacial bonding between the filler and resin matrix and improves mechanical properties. Both micrometer size and nanometer size fillers can be used in compositions, with fumed silica being exemplary, The filler amounts may be between 0 and 80 parts by weight, for example between 0 and 40 parts by weight. In one embodiment, the filler is present in an amount greater than 1 part by weight, for example, greater than 5 parts by weight. In another embodiment, the filler is present in an amount less than 80 parts by weight, for example, less than 30 parts by weight.

Fluoride releasing and anti-microbial agents can also be incorporated in compositions to prevent secondary caries and reduce plaque formation. The fluoride releasing agents may be fillers or ethylenically unsaturated monomers. The fluoride releasing fillers include, but are not limited to, sodium hexafluorosilicate, zinc hexafluorosilicate, strontium hexafluorosilicate, sodium fluoride, potassium fluoride, calcium fluoride, strontium fluoride, magnesium fluoride and water-soluble inorganic complex fluoride described in U.S. Pat. No. 5,824,720 (Nowak et al.). Ethylenically unsaturated fluoride releasing agents include, but are not limited to, fluoride-containing complexes of Lewis bases and boron trifluoride described in U.S. Pat. No. 4,772,325 (Kwan and O'Connell) and chelating monomers described in U.S. Pat. No. 6,703,5 8 (Xu et al.). Anti-microbial agents include, but are not limited to, benzalkonium chloride, triclosan, alkyl 4-hydroxybenzoate, silicate glass powder containing silver and/or zinc, and zeolite powder containing silver and/or zinc. Examples of commercially available antimicrobial additives are Irgaguard B 1000 (triclosan), Irgaguard B 5000 (silver-zinc zeolite) and B 6000 (silver glass / zeolite) and 7000 (silver-zinc glass). All Irgaguard products are from Ciba Specialty Chemicals.

To obtain a chemically stable composition that has a shelf-life of at least one year and preferably two years at normal room temperature, a stabilizer may be included in the composition. These compounds include, but are not limited to, 3,5-di-tert-butyl-4-hydroxytoluene (BHT) and hydroquinone monomethyl ether (MEHQ). The concentration for the stabilizer may be between 0.0001 and 5% by weight, for example, greater than 0.01% or greater than 0.1%, and by further example, less than 4% or less than 3%.

Colors can be measured by several measuring systems, one of which is the L, a, b color scale. The L, a, b color scale is organized in a cube form. The L axis runs from top to bottom, with a maximum value of 100 (perfect reflecting diffuser) and a minimum value of zero (black), The a axis represents red and green, with a positive value for red and a negative value for green. The b axis represents yellow and blue, with a positive value for yellow and a negative value for blue.

In one embodiment, the color change (ΔE) during the curing of the mixed composition is at least 5. In one embodiment, the color change (ΔE) during the curing of the mixed composition is at least 20. In one embodiment, the color change (ΔE) during the curing of the mixed composition is at least 25. In one embodiment, the color change (ΔE) during the curing of the mixed composition is at least 30.

### EXAMPLES

In the examples, the following materials were used:
- BHT:: 2,6-di-(tert-butyt)-4-methylphenol
- Bis-GMA:: 2,2-bis[4-(2-hydroxy-3-methacryloylpropoxy)-phenyl]-propane
- CHPO:: cumene hydroperoxide
- GDM:: glycerol dimethacrylate
- GPDM:: glycerolphosphate dimethacrylate
- HEMA:: hydroxyethyl methacrylate
- MEHQ:: 4-methoxyphenol
- OX-50:: fumed silica or colloidal silica (from Degussa)
- PMR:: penta-methoxy red
- PTU:: 1-(2-pyridyl)-2-thiourea
- RG:: reactive (ionomer) dental glass, not surface treated
- SR454:: ethoxylated (3) trimethylolpropane triacrylate
- SRG:: silane-treated reactive (ionomer) dental glass
- ST-BAS:: bariumaluminoborosilicate filler, mean particle size of 1.0 µm, and surface-treated with γ-methacryloyloxypropyltrimethoxysilane.
- ST-OX50:: OX-50 surface-treated with γ-methacryloyloxypropyltrimethoxysilane
- TS-530:: surface treated fumed silica or colloidal silica (from Cabot Corp.)

Dental resins were made by mixing all chemicals indicated in Table 1. Dental pastes were made by adding solid fillers into corresponding resins, as shown in Table 2. All pastes were made by mixing ingredients according to the ratios, followed by milling to disperse the fillers.

**Table 1. Compositions of resins in wt.%**

| **Resin number** | **RA1** | **RA2** | **RA3** | **RA4** | **RB1** | **RB2** | **RB3** | **RB4** |
|---|---|---|---|---|---|---|---|---|
| GPDM | 19.91 | 19.92 | 19.93 | 14.64 | | | | |
| BisGMA | 24.89 | 24.9 | 24.91 | 24.4 | 41.88 | 41.9 | 41.92 | 49.21 |
| HEMA | 43.81 | 43.83 | 43.85 | 29.28 | 9.97 | 9.98 | 9.98 | 24.61 |
| GDM | | | | 19.52 | 24.93 | 24.94 | 24.95 | 24.61 |
| SR454 | | | | | 21.94 | 21.94 | 21.95 | |
| CHPO | 3.19 | 3.19 | 3.19 | 2.25 | | | | |
| PTU | | | | | 1.2 | 1.2 | 1.2 | 1.47 |
| PMR | 0.08 | 0.04 | | | 0.08 | 0.04 | | 0.06 |
| BHT | 0.15 | 0.15 | 0.15 | 0.15 | | | | |
| MEHQ | | | | | | | | 0.04 |
| Water | 7.97 | 7.97 | 7.97 | 9.76 | | | | |

**Table 2. Compositions of pastes in wt.%**

| **Paste number** | **A1** | **A2** | **A3** | **A4** | **B1** | **B2** | **B3** | **B4** | **B5** |
|---|---|---|---|---|---|---|---|---|---|
| | **RA1** | **RA2** | **RA3** | **RA4** | **RB1** | **RB2** | **RB3** | **RB4** | **RB4** |
| Resins | 70 | 70 | 70 | 35 | 28 | 28 | 28 | 37 | 35 |
| ST-OX50 | 30 | 30 | 30 | | | | | | |
| TS-530 | | | | 1 | 3 | 3 | 3 | 1 | 1 |
| SRG | | | | | 69 | 69 | 69 | | 64 |
| RG | | | | | | | | 62 | |
| ST-BAS | | | | 64 | | | | | |

### Example 1

The paste A1 was mixed with the paste B3 at a ratio of 1:1. The mixing was finished by a centrifuge at a speed of about 3000 RPM (rotations per minute) for 15 seconds. The mixed paste had a color of deep purple. The paste was placed into a metal mold of 1 mm in thickness and 20 mm in diameter. The mixture cured to a hard disc in 10 minutes. The color of the paste at this point was deep purple. The purple color became significantly lighter after 1 day in 37°C water and nearly disappeared (with very light purple color) after 3 days in 37°C water. Color was measured using the L, a and b system against time (10 min, I hour, 1 day and 3 days), and the results are set forth in Table 3.

**Table 3. Color measurements of the mixture of A1 and B3 (1:1 ratio)**

| | 10 min | 1h | 1 day | 3 days |
|---|---|---|---|---|
| L | 22.72 | 22.95 | 43.26 | 54.33 |
| a | 7.25 | 7.54 | 7.28 | 10.98 |
| b | -3.51 | -3,87 | -5.27 | 8.33 |

### Example 2

The paste A2 was mixed with the paste B3 at a ratio of 1:1. The mixing was finished by a centrifuge at a speed of about 3000 RPM for 15 seconds, The mixed paste had a color of deep purple. The paste was placed into a metal mold of 1 mm in thickness and 20 mm in diameter. The mixture cured to a hard disc in 10 minutes. The color of the paste at this point was deep purple. The purple color became significantly lighter after 1 day in 37°C water and nearly disappeared after 3 days in 37°C water. Color was measured using an L, a and b system against time (10 min, I hour, 1 day and 3 days), and the results are set forth in Table 4.

**Table 4. Color measurements of the mixture of A2 and B3 (1:1 ratio)**

| | 10 min | 1h | 1 day | 3 days |
|---|---|---|---|---|
| L | 25.34 | 27.67 | 50.27 | 66.3 |
| a | 14.06 | 12.79 | 10.16 | 6.67 |
| b | -6.09 | -6.4 | 1.42 | 15.37 |

### Example 3

The paste A3 was mixed with the paste B1 at a ratio of 1:1. The mixing was finished by a centrifuge at a speed of about 3000 RPM for 15 seconds. The mixed paste had a color of deep purple. The paste was placed into a metal mold of 1 mm in thickness and 20 mm in diameter. The mixture cured to a hard disc in 10 minutes. The color of the paste at this point was deep purple. The purple color became significantly lighter after 1 day in 37°C water and nearly disappeared (with very light purple color) after 3 days in 37°C water. Color was measured using an L, a and b system against time (10 min, 1 hour, 1 day and 3 days), and the results are set forth in Table 5.

**Table 5. Color measurements of the mixture of A3 and B1 (1:1 ratio)**

| | 10 min | 1h | 1 day | 3 days |
|---|---|---|---|---|
| L | 26.84 | 27.58 | 50.78 | 70.18 |
| a | 12.09 | 12.15 | 9.68 | 3.42 |
| b | -7.24 | -8.63 | -11.26 | 4.39 |

### Example 4

The paste A3 was mixed with the paste B2 at a ratio of 1:1. The mixing was finished by a centrifuge at a speed of about 3000 RPM for 15 seconds. The mixed paste had a color of deep purple. The paste was placed into a metal mold of 1 mm in thickness and 20 mm in diameter. The mixture cured to a hard disc in 10 minutes. The color of the paste at this point was deep purple. The purple color became significantly lighter after 1 day in 37°C water and nearly disappeared after 3 days in 37°C water. Color was measured using an L, a and b system against time (10 min, 1 hour, 1 day and 3 days), and the results are set forth in Table 6.

**Table 6. Color measurements of the mixture of A3 and B2 (1:1 ratio)**

| | 10 min | 1h | 1 day | 3 days |
|---|---|---|---|---|
| L | 33.08 | 34.34 | 62.03 | 77.73 |
| a | 16.56 | 16.04 | 7.89 | 0.45 |
| b | -12.36 | -12.72 | -7.88 | 4.14 |

### Example 5

The paste A4 was mixed with the paste B4 at a ratio of 4:1. The mixed paste (through a static mixer) had a medium purple color. The paste was placed into a metal mold of 1 mm in thickness and 20 mm in diameter. The mixture cured to a hard disc in 10 minutes. The color of the paste at this point was medium purple. The purple color disappeared after 1 day in 37°C water. Color was measured using an L, a and b system against time (10 min, 1 hour, and 1 day), and the results are set forth in Table 7.

**Table 7. Color measurements of the mixture of A4 and B4 (4:1 ratio)**

| | 10 min | 1h | 1 day |
|---|---|---|---|
| L | 71.70 | 72.64 | 77.37 |
| A | 3.07 | 2.62 | 0.21 |
| B | -0.96 | -0.04 | 2.89 |

### Example 6

The pastes A4 was mixed with the paste B5 at a ratio of 4:1. The mixed paste (through a static mixer) had a medium purple color. The paste was placed into a metal mold of I mm in thickness and 20 mm in diameter. The mixture cured to a hard disc in 10 minutes. The color of the paste at this point was medium purple. The purple color disappeared after 1 day in 37°C water. Color was measured using an L, a and b system against time (10 min, 1 hour, and 1 day), and the results are set forth in Table 8.

**Table 8. Color measurements of the mixture of A4 and B5 (4:1 ratio)**

| | 10 min | 1h | 1 day |
|---|---|---|---|
| L | 67.27 | 70.91 | 76.82 |
| A | 4.84 | 3.56 | 0.56 |
| B | -2.68 | -0.32 | 2.90 |

The compositions thus exhibit a visually observable purple color upon mixing, which color changes over time as the pH changes as a result of the penta-methoxy red color change agent, and the color essentially disappears after 1-3 days. This color change allows the practitioner to observe full mixing of the components, and to clearly see where the composition is applied within the oral environment so that the restoration can be shaped and excess material removed where necessary, yet the color dissipates so that the patient is left with no visible color in the restoration after a short period of time.

While the present invention has been illustrated by the description of one or more embodiments thereof, and while the embodiments have been described in considerable detail, additional advantages and modifications will readily appear to those skilled in the art. The invention in its broader aspects is therefore not limited to the specific details, representative apparatus and method and illustrative examples shown and described.

## Claims

1. A dental composition that is capable of undergoing a color change, comprising a first part containing an acidic component and a second part containing a basic component, **characterized in that** at least one color change agent is included in the first and/or the second part that effects a change in color in the dental composition upon an increase or decrease in pH.

2. The dental composition of claim 1 wherein the basic component is a dental filler.

3. The dental composition of either claim 1 or claim 2 wherein the acidic component includes at least one acidic polymer.

4. The dental composition of any preceding claim wherein the acidic component includes at least one carboxylic acid, phosphoric acid, or phosphate with at least one ethylenically unsaturated group.

5. The dental composition of any preceding claim wherein the at least one color change agent is penta-methoxy red.

6. The dental composition of any preceding claim wherein the change in color is from a visually observable color to essentially colorless.

7. The dental composition of any preceding claim wherein the first part and/or the second part includes at least one neutral monomer with at least one ethylenically unsaturated group.

8. The composition of any preceding claim wherein the change in color (ΔE) is at least 20.

9. The dental composition of any preceding claim, wherein:
mixing the first part containing the acidic component with the second part containing the basic component forms a mixed dental composition in which a neutralization reaction takes place, wherein the at least one color change agent that is acid sensitive effects a change in color in the mixed dental composition upon an increase in pH from the neutralization reaction, as or after the mixed dental composition is applied to a dental structure.

10. The composition of claim 9 wherein the change in color occurs after the mixed dental composition is applied to a dental structure.

11. The dental composition of any one of claims I to 8, wherein the first part contains the acidic component, water and the at least one color change agent that is acid sensitive and wherein the first part is self-etching and/or self-adhesive to a dental structure, wherein the second part is non-self-etching and non-self-adhesive to a dental structure and initiates a neutralization reaction with the acidic component in the first part, when the second part containing the basic component is applied in contact with the first part present on the dental structure, and wherein the at least one acid sensitive color change agent effects a change in color in the first dental composition upon an increase in pH from the neutralization reaction.

12. A method of using the dental composition of any one of claims 1 to 8, comprising:
mixing the first part containing the acidic component with the second part containing the basic component to for a mixed dental composition in which a neutralization reaction takes place, wherein the at least one color change agent that is acid sensitive effects a change in color in the mixed dental composition upon an increase in pH from the neutralization reaction, as or after the mixed dental composition is applied to a dental structure.

13. The method of claim 12 wherein the change in color occurs after the mixed dental composition is applied to a dental structure.

14. A method of using the dental composition of any one of claims 1 to 8, wherein the first part contains the acidic component, water and the at least one color change agent that is acid sensitive and wherein the first part is self-etching and/or self-adhesive to a dental structure, the method comprising applying the second part containing the basic component in contact with the first part present on a dental structure, wherein the second part is non-self-etching and non-self-adhesive to the dental structure and initiates a neutralization reaction with the acidic component in the first part, and wherein the at least one acid sensitive color change agent effects a change in color in the first dental composition upon an increase in pH from the neutralization reaction.
